# EUROPEAN PATENT SPECIFICATION

(11) **EP 1 880 012 B1**
(45) Date of publication and mention of the grant of the patent: **08.07.2015**
(21) Application number: 06743016.5
(22) Date of filing: 10.05.2006
(51) Int. Cl.: C12N 15/861, A61K 39/21, A61K 35/76, C07K 14/16

(54) **VACCINE COMPOSITION**
IMPFSTOFFZUSAMMENSETZUNG
COMPOSITION DE VACCIN

(30) Priority: 12.05.2005 US 680389 P
(43) Date of publication of application: 23.01.2008
(73) Proprietor: Glaxo Group Limited, Brentford, Middlesex TW8 9GS (GB)
(72) Inventor: ERTL, Peter, Franz GlaxoSmithKline, Stevenage Hertfordshire SG1 2NY (GB); TITE, John, Philip GlaxoSmithKline, Stevenage Hertfordshire SG1 2NY (GB); VAN WELY, Catherine Ann GlaxoSmithKline, Stevenage Hertfordshire SG1 2NY (GB)
(74) Representative: Fowler, Gavin James
(86) International application number: PCT/EP2006/004854
(87) International publication number: WO 2006/120034

(56) References cited:
- WO-A-02/22080
- WO-A-03/025003
- WO-A-2004/041851
- WO-A-2004/041852
- WO-A-2006/013106
- GB-A- 2 406 336
- FITZGERALD J C ET AL: "A Simian Replication-Defective Adenoviral Recombinant Vaccine to HIV-1 Gag" JOURNAL OF IMMUNOLOGY, THE WILLIAMS AND WILKINS CO. BALTIMORE, US, vol. 170, February 2003 (2003-02), pages 1416-1422, XP002974900 ISSN: 0022-1767

## Description

### FIELD OF THE INVENTION

The present invention relates to virus vectors comprising oligonucleotides encoding HIV polypeptides, more particularly wherein the virus vector is an adenovirus. In particular, such adenoviruses are non-human primate adenoviruses such as simian adenoviruses, more particularly chimpanzee adenoviruses. In particular the invention relates to adenovirus vectors which comprise HIV polynucleotide sequences which encode multiple different HIV antigens, for example two or three or more HIV antigens. The invention further relates to methods of preparing the virus vectors, to the virus vectors produced by the methods and to the use of the vectors in medicine especially prophylactic or therapeutic vaccination.

HIV-1 is the primary cause of the acquired immune deficiency syndrome (AIDS) which is regarded as one of the world's major health problems. Although extensive research throughout the world has been conducted, efforts to produce a vaccine thus far have not been successful.

HIV-1 is an RNA virus of the family Retroviridiae. The HIV genome encodes at least nine proteins which are divided into three classes: the major structural proteins Gag, Pol and Env, the regulatory proteins Tat and Rev, and the accessory proteins Vpu, Vpr, Vif and Nef. The HIV genome exhibits the 5'LTR-gag-pol-env-LTR3' organization of all retroviruses.

Adenovirus is a double-stranded DNA virus with a genome size of about 36 kb, which has been widely used for gene transfer applications due to its ability to achieve highly efficient gene transfer in a variety of target tissues and large transgene capacity. Conventionally, E1 genes of adenovirus are deleted and replaced with a transgene cassette consisting of the promoter of choice, cDNA sequence of the gene of interest and a polyA signal, resulting in a replication defective recombinant virus.

Adenoviruses have a characteristic morphology with an icosohedral capsid consisting of three major proteins, hexon (II), penton base (III) and a knobbed fibre (IV), along with a number of other minor proteins, VI, VIII, IX, IIIa and IVa2 (Russell W.C. 2000, Gen Virol, 81:2573-2604). The virus genome is a linear, double-stranded DNA with a terminal protein attached covalently to the 5' termini, which have inverted terminal repeats (ITRs). The virus DNA is intimately associated with the highly basic protein VII and a small peptide termed mu. Another protein, V, is packaged with this DNA-protein complex and provides a structural link to the capsid via protein VI. The virus also contains a virus-encoded protease, which is necessary for processing of some of the structural proteins to produce mature infectious virus.

Over 100 distinct serotypes of adenovirus halve been isolated which infect various mammalian species, 51 of which are of human origin. Examples of such adenoviruses from human origin are Ad1, Ad2, Ad4, Ad5, Ad6, Ad11, Ad 24, Ad34, Ad35. The human serotypes have been catagorised into six subgenera (A-F) based on a number of biological, chemical, immunological and structural criteria. [page 1, WO04018627]

Although Ad5-based vectors have been used extensively in a number of gene therapy trials, there may be limitations on the use of Ad5 and other group C adenoviral vectors due to preexisting immunity in the general population due to natural infection. Ad5 and other group C members tend to be among the most seroprevalent serotypes. Immunity to existing vectors may develop as a result of exposure to the vector during treatment. These types of preexisting or developed immunity to seroprevalent vectors may limit the effectiveness of gene therapy or vaccination efforts. Alternative adenovirus serotypes, thus constitute very important targets in the pursuit of gene delivery systems capable of evading the host immune response.

One such area of alternative serotypes are those of non human primates, especially chimpanzee adenoviruses. See US Patent 6,083,716 which describes the genome of two chimpanzee adenoviruses.

It has been shown that chimpanzee ("Pan" or "C") adenoviral vectors induce strong immune responses to transgene products as efficiently as human adenoviral vectors (Fitzgerald et al. J. Immunol. 170:1416).

HIV Tat and Nef proteins are early proteins, that is, they are expressed early in infection and in the absence of structural protein.

The Nef gene encodes an early accessory HIV protein which has been shown to possess several activities. For example, the Nef protein is known to cause the removal of CD4, the HIV receptor, from the cell surface, although the biological importance of this function is debated. Additionally Nef interacts with the signal pathway of T cells and induces an active state, which in turn may promote more efficient gene expression. Some HIV isolates have mutations in this region, which cause them not to encode functional protein and are severely compromised in their replication and pathogenesis in vivo.

The Gag gene is translated from the full-length RNA to yield a precursor polyprotein which is subsequently cleaved into 3 - 5 capsid proteins; the matrix protein, capsid protein and nucleic acid binding protein and protease. (Fundamental Virology, Fields BN, Knipe DM and Howley M 1996 2. Fields Virology vol 2 1996).

The Gag gene gives rise to the 55-kilodalton (kD) Gag precursor protein, also called p55, which is expressed from the unspliced viral mRNA. During translation, the N terminus of p55 is myristoylated, triggering its association with the cytoplasmic aspect of cell membranes. The membrane-associated Gag polyprotein recruits two copies of the viral genomic RNA along with other viral and cellular proteins that triggers the budding of the viral particle from the surface of an infected cell. After budding, p55 is cleaved by the virally encoded protease (a product of the Pol gene) during the process of viral maturation into four smaller proteins designated MA (matrix [p17]), CA (capsid [p24]), NC (nucleocapsid [p9]), and p6.(4).

In addition to the 3 major Gag proteins (p17, p24 and p9), all Gag precursors contain several other regions, which are cleaved out and remain in the virion as peptides of various sizes. These proteins have different roles e.g. the p2 protein has a proposed role in regulating activity of the protease and contributes to the correct timing of proteolytic processing.

The MA polypeptide is derived from the N-terminal, myristoylated end of p55. Most MA molecules remain attached to the inner surface of the virion lipid bilayer, stabilizing the particle. A subset of MA is recruited inside the deeper layers of the virion where it becomes part of the complex which escorts the viral DNA to the nucleus. These MA molecules facilitate the nuclear transport of the viral genome because a karyophilic signal on MA is recognized by the cellular nuclear import machinery. This phenomenon allows HIV to infect non-dividing cells, an unusual property for a retrovirus.

The p24 (CA) protein forms the conical core of viral particles. Cyclophilin A has been demonstrated to interact with the p24 region of p55 leading to its incorporation into HIV particles. The interaction between Gag and cyclophilin A is essential because the disruption of this interaction by cyclosporin A inhibits viral replication.

The NC region of Gag is responsible for specifically recognizing the so-called packaging signal of HIV. The packaging signal consists of four stem loop structures located near the 5' end of the viral RNA, and is sufficient to mediate the incorporation of a heterologous RNA into HIV-1 virions. NC binds to the packaging signal through interactions mediated by two zinc-finger motifs. NC also facilitates reverse transcription.

The p6 polypeptide region mediates interactions between p55 Gag and the accessory protein Vpr, leading to the incorporation of Vpr into assembling virions. The p6 region also contains a so-called late domain which is required for the efficient release of budding virions from an infected cell.

The Pol gene encodes three proteins having the activities needed by the virus in early infection, reverse transcriptase RT, protease, and the integrase protein needed for integration of viral DNA into cellular DNA. The primary product of Pol is cleaved by the virion protease to yield the amino terminal RT peptide which contains activities necessary for DNA synthesis (RNA and DNA directed DNA polymerase, ribouclease H) and carboxy terminal integrase protein. HIV RT is a heterodimer of full-length RT (p66) and a cleavage product (p51) lacking the carboxy terminal Rnase integrase domain.

RT is one of the most highly conserved proteins encoded by the retroviral genome. Two major activities of RT are the DNA Pol and Ribonuclease H. The DNA Pol activity of RT uses RNA and DNA as templates interchangeably and like all DNA polymerases known is unable to initiate DNA synthesis de novo, but requires a pre existing molecule to serve as a primer (RNA).

The Rnase H activity inherent in all RT proteins plays the essential role early in replication of removing the RNA genome as DNA synthesis proceeds. It selectively degrades the RNA from all RNA - DNA hybrid molecules. Structurally the polymerase and ribo H occupy separate, non-overlapping domains within the Pol covering the amino two thirds of the Pol.

The p66 catalytic subunit is folded into 5 distinct subdomains. The amino terminal 23 of these have the portion with RT activity. Carboxy terminal to these is the Rnase H Domain.

After infection of the host cell, the retroviral RNA genome is copied into linear ds DNA by the reverse transcriptase that is present in the infecting particle. The integrase (reviewed in Skalka AM '99 Adv in Virus Res 52 271-273) recognises the ends of the viral DNA, trims them and accompanies the viral DNA to a host chromosomal site to catalyse integration. Many sites in the host DNA can be targets for integration. Although the integrase is sufficient to catalyse integration in vitro, it is not the only protein associated with the viral DNA in vivo - the large protein - viral DNA complex isolated from the infected cells has been denoted the pre integration complex. This facilitates the acquisition of the host cell genes by progeny viral genomes.

The integrase is made up of 3 distinct domains, the N terminal domain, the catalytic core and the C terminal domain. The catalytic core domain contains all of the requirements for the chemistry of polynucleotidyl transfer.

Virus vectors and particularly adenovirus vectors containing multiple foreign genes are not always easy to produce. There may be problems with the stability of the vectors, and difficulties with getting effective expression of the inserted genes. In particular, adenoviruses containing more than one or more than two HIV polynucleotides that could be used in a vaccine have not been successfully produced.

Non human primate adenoviruses can be isolated from the mesenteric lymph nodes of chimpanzees. Chimpanzee adenoviruses are sufficiently similar to human adenovirus subtype C to allow replication of E1 deleted virus in HEK 293 cells. Yet chimpanzee adenoviruses are phylogenetically distinct from the more common human serotypes (Ad2 and Ad5). Pan 6 is less closely related to and is serologically distinct from Pan's 5, 7 and 9.

There are certain size restrictions associated with inserting heterologous DNA into adenoviruses. Human adenoviruses have the ability to package up to 105% or the wild type genome length (Bett el at 1993, J Virol 67(10), 5911-21). The lower packaging limit for human adenoviruses has been shown to be 75% of the wild type genome length (Parks et al 1995, J Virol 71(4), 3293-8)

There is still a need to find an effective vaccine against HIV.

The present invention provides an adenovirus vector comprising a polynucleotide or polynucleotides encoding at least HIV antigens RT, Net and Gag or immunogenic fragments thereof arranged so that they are transcribed in the order Gag, RT, Net, so that the Gag portion is at the N terminal end of the resulting fusion protein, characterized in that the virus is a non-human primate adenovirus, and the virus is a chimpanzee adenovirus selected from Pan 5, Pan 6 and Pan 7.

Provided is an adenovirus vector deleted in one or more regions, which vector comprises a polynucleotide or polynucleotides encoding at least three HIV antigens or immunogenic fragments thereof wherein the vector is capable of expressing the antigens or fragments or derivatives in a mammalian host and wherein the size of the deletion and the size of the HIV polynucleotide or polynucleotides are such that the overall length of the vector genome is between 85 and 105% of the length of the wild type virus genome.

In one embodiment the HIV antigens encoded by the polynucleotide or polynucleotides may be Gag, Nef and Pol. In a further embodiment, Pol may comprise the RT portion only. In yet another embodiment the polynucleotide or polynucleotides encoding the HIV antigens may be arranged so that they are transcribed in the order Gag, RT, Nef, I.e. so that the Gag portion is at the N-terminal end of the resulting fusion protein.

The size of the overall vector genome may be for example from 90 to 100% of the size of the wild type virus genome, or from 95 to 100% of the size of the wild type genome. The overall size of the vector may be about 96% of the size of the wild type virus genome.

Particular HIV antigens for inclusion in the adenovirus vectors are Pol, Nef and Gag or immunogenic fragments thereof.

Such adenovirus vectors may be formulated with pharmaceutically acceptable excipient, carriers, diluents or adjuvants to produce immunogenic compositions including pharmaceutical or vaccine compositions suitable for the treatment and/or prophylaxis of HIV infection and AIDS.

Of use are adenoviruses which are distinct from prevalent naturally occurring serotypes in the human population such as Ad2 and Ad5. This avoids the induction of potent immune responses against the vector which limits the efficacy of subsequent administrations of the same serotype by blocking vector uptake through neutralizing antibody and influencing toxicity.

Thus, the adenovirus may be an adenovirus which is not a prevalent naturally occurring human virus serotype. Adenoviruses isolated from animals have immunologically distinct capsid, hexon, penton and fibre components but are phylogenetically closely related. Specifically, the virus may be a non-human adenovirus, such as a simian adenovirus and in particular a chimpanzee adenovirus such as Pan 5, 6, 7 or 9. Examples of such strains are described in WO03/000283 and are available from the American Type Culture Collection, 10801 University Boulevard, Manassas, Virginia 20110-2209, and other sources. Desirable chimpanzee adenovirus strains are Pan 5 [ATCC VR-591], Pan 6 [ATCC VR-592], and Pan 7 [ATCC VR-593]. Other suitable adenoviruses include, without limitation, chimpanzee adenoviruses C1 and C68 (Pan9), described in US Patent No. 6,083,716; and simian adenoviruses including, without limitation SV1 [VR-195]; SV25 [SV-201]; SV35; SV15; SV-34; SV-36; SV-37, and baboon adenovirus [VR-275], among others. The sequences of Pan 5 (also termed C5), Pan 6 (also termed C6), Pan 7 (also termed C7), SV1, SV25, and SV39 have been described [WO 03/046124, published 5 June 2003]. See, also, International Patent Publication No. WO 04/16614, which describes hybrid adenovirus vectors and vectors constructed from simian adenovirus SA18.

Chimpanzee adenoviruses are thought to be advantageous over human adenovirus serotypes because of the lack of pre-existing immunity, in particular the lack of cross-neutralising antibodies, to adenoviruses in the target population. Cross-reaction of the chimpanzee adenoviruses with pre-existing neutralizing antibody responses is only present in 2% of the target population compared with 35% in the case of certain candidate human adenovirus vectors. The chimpanzee adenoviruses are distinct from the more common human subtypes Ad2 and Ad5, but are more closely related to human Ad4 of subgroup E, which is not a prevalent subtype. Pan 6 is less closely related to Pan 5, 7 and 9.

The adenovirus may be replication defective. This means that it has a reduced ability to replicate in non-complementing cells, compared to the wild type virus. This may be brought about by mutating the virus e.g. by deleting a gene involved in replication, for example deletion of the E1a, E1b, E3 or E4 gene.

The adenovirus vectors may be replication defective adenovirus comprising a functional E1 deletion. Thus the adenovirus vectors may be replication defective due to the absence of the ability to express adenoviral E1a and E1b, i.e., are functionally deleted in E1a and E1b. The recombinant adenoviruses may also bear functional deletions in other genes [see WO 03/000283] for example, deletions in E3 or E4 genes. The adenovirus delayed early gene E3 may be eliminated from the simian adenovirus sequence which forms part of the recombinant virus. The function of E3 is not necessary to the production of the recombinant adenovirus particle. Thus, it is unnecessary to replace the function of this gene product in order to package a recombinant simian adenovirus useful in the invention. In one particular embodiment the recombinant (simian) adenoviruses have functionally deleted E1 and E3 genes. The construction of such vectors is described in Roy et al., Human Gene Therapy 15:519-530, 2004.

Recombinant adenoviruses may also be constructed having a functional deletion of the E4 gene, although it may be desirable to retain the E4 ORF6 function. Adenovirus vectors may also contain a deletion in the delayed early gene E2a. Deletions may also be made in any of the late genes L1 through to L5 of the simian adenovirus genome. Similarly deletions in the intermediate genes IX and IVa may be useful.

Other deletions may be made in the other structural or non-structural adenovirus genes. The above deletions may be used individually, i.e. an adenovirus sequence for use in the present invention may contain deletions of E1 only. Alternatively, deletions of entire genes or portions thereof effective to destroy their biological activity may be used in any combination. For example in one exemplary vector, the adenovirus sequences may have deletions of the E1 genes and the E4 gene, or of the E1, E2a and E3 genes, or of the E1 and E3 genes (such as functional deletions in E1a and E1b, and a deletion of at least part of E3), or of the E1, E2a and E4 genes, with or without deletion of E3 and so on. Such deletions may be partial or full deletions of these genes and may be used in combination with other mutations, such as temperature sensitive mutations to achieve a desired result.

The adenoviral vectors can be produced on any suitable cell line in which the virus is capable of replication. In particular, complementing cell lines which provide the factors missing from the virus vector that result in its impaired replication characteristics can be used. For example, a complementing cell ling may express E1, or E1 and E3, or E1, E3 and E4. Without limitation, such a cell line may be HeLa [ATCC Accession No. CCL 2], A549 [ATCC Accession No. CCL 185], HEK 293, KB [CCL 17] Detroit [e.g., Detroit 510, CCL 72] and WI-38 [CCL 75] cells, among others. These cell lines are all available from the American Type Culture Collection, 10801 University Boulevard, Manassas, Virginia 20110-2209. Other suitable parent cell lines may be obtained from other sources, such as PER.C6© cells, as represented by the cells deposited under ECACC no. 96022940 at the European Collection of Animal Cell Cultures (ECACC) at the Centre for Applied Microbiology and Research (CAMR, UK).

Provided, in another aspect an adenovirus vector comprising a polynucleotide or polynucleotides encoding at least HIV antigens RT, Nef and Gag or immunogenic fragments thereof in the order Gag, RT, Nef, that is to say an adenovirus vector comprising a polynucleotide or polynucleotides encoding at least HIV antigens RT, Nef and Gag or immunogenic fragments thereof arranged so that they are transcribed In the order Gag, RT, Nef.

For example an adenovirus vector according to the invention may comprise a polynucleotide encoding Gag or an immunogenic derivative or immunogenic fragment thereof, fused to a polynucleotide sequence encoding RT or an immunogenic derivative or immunogenic fragment thereof, fused to Nef or an immunogenic derivative or immunogenic fragment thereof, and under the control of a single heterologous promoter, wherein the Gag portion of the gene is present on the 5' terminus of the polynucleotide.

In an alternative embodiment, each of the three antigens is expressed through its own promoter, each of said promoters may be the same or different. In yet another embodiment of the invention two of the three antigens form a fusion, linked to a single promoter and the third antigen is linked to a second promoter, which may be the same or different from the first promoter. For example, Gag and RT may be linked to a first promoter and Nef may be linked to a second promoter.

The polynucleotide or polynucleotides encoding at least three HIV antigens or immunogenic fragments thereof may be inserted into any of the Adeno deleted regions, for example into the E1 deleted region.

Although two or more polynucleotides encoding antigens may be linked as a fusion, the resulting protein may be expressed as a fusion protein, or it may be expressed as separate protein products, or it may be expressed as a fusion protein and then subsequently broken down into smaller subunits.

In one aspect, the present invention provides a fusion protein expressed by a vector according to the invention, for example, a fusion protein produced within the human body.

One or more of the HIV sequences included in the vector according encoding e.g Nef, Gag or RT may be codon optimised for mammalian cells, for example such that it/they resemble a highly expressed human gene in their codon use. Codon optimization of these HIV sequences is further described in WO 03/025003.

For example, the polynucleotides encoding Gag and/or RT in the adenovirus vectors may be codon optimised as discussed above.

The Gag sequence in the adenovirus vector may exclude the Gag p6 polypeptide encoding sequence. A particular example of a Gag sequence for use in the invention comprises p17 and/or p24 encoding sequences.

The RT sequence may encode a mutation to substantially inactivate any reverse transcriptase activity. One particular inactivation mutation involves the substitution of W tryptophan 229 for K (lysine), see WO03/025003.

The RT gene is a component of the bigger Pol gene in the HIV genome as described above. It will be understood that the RT encoding sequence included in the adenovirus vector according to the invention may be present in the context of Pol, or a fragment of Pol encoding at least RT. Such fragments of Pol retain major CTL epitopes of Pol. In one specific example, RT is included as just the p51 or just the p66 fragment of RT.

Optionally the Nef sequence for use is truncated to remove the sequence encoding the N terminal region i.e. removal of from 30 to 85 amino acids, for example from 60 to 85 amino acids, particularly the N terminal 65 amino acids (the latter truncation is referred to herein as trNef). Alternatively or additionally the Nef may be modified to remove one or more myristylation sites. For example the Gly 2 myristylation site may be removed by deletion or substitution. Alternatively or additionally the Nef may be modified to alter the dileucine motif of Leu 174 and Leu 175 by deletion or substitution of one or both leucines. The importance of the dileucine motif in CD4 downregulation is described e.g. in Bresnahan P.A. et al (1998) Current Biology, 8(22): 1235-8.

A construct may comprise Gag, Pol and Nef wherein at least 75%, or at least 90% or at least 95%, for example, 96% of the CTL epitopes of these native antigens are present.

In a construct which comprises p17/p24 Gag, p66 RT, and truncated Nef as defined above, 96% of the CTL epitopes of the native Gag Pol and Nef antigens are present.

One embodiment provides an adenovirus vector comprising a polynucleotide or polynucleotides encoding p17, p24 (optimized) Gag, p66 RT (optimised), truncated Nef (devoid of nucleotides encoding terminal amino-acids 1-85 - "trNef') in the order Gag, RT, Nef.

Constructs include:
1. p17, p24 (codon optimised) Gag - p66 RT (codon optimised) - truncatedNef;
2. truncatedNef - p66 RT (codon optimised) - p17, p24 (codon optimised) Gag;
3. truncatedNef - p17, p24 (codon optimised) Gag - p66 RT (codon optimised);
4. p66 RT (codon optimised) - p17, p24 (codon optimised) Gag - truncatedNef;
5. p66 RT (codon optimised) - truncatedNef - p17, p24 (codon optimised) Gag;
6. p17 , p24 (codon optimised) Gag - truncatedNef - p66 RT (codon optimised).

The polynucleotide or polynucleotides may have linker sequences present in between the sequences encoding Gag, RT and Nef. Such linker sequences may be, for example, up to 20 amino acids in length. In a particular example they may be from 1 to 10 amino acids, or from 1 to 6 amino acids, for example 2 to 4 amino acids.

The polynucleotides may contain further HIV sequences. In particular, they may include HIV env proteins or immunogenic fragments thereof. Suitable forms of env are gp120, gp140 and gp160. Other suitable HIV sequences include but are not limited to Tat, Rev, Vpu, Vpr and Vif. Further provided is an adenovirus vector comprising a polynucleotide or polynucleotides encoding HIV antigens RT, Nef and Gag or immunogenic derivatives or immunogenic fragments thereof in the order Gag, RT, Nef, together with an HIV env protein or immunogenic derivative or immunogenic fragment thereof.

The present invention furthermore comprises an immunogenic composition comprising an adenoviral vector according to the present invention in combination with a second adenoviral vector comprising a polynucleotide or polynucleotides encoding one or more HIV antigens.

It will be understood that for all of the HIV sequences included, these do not necessarily represent sequences encoding the full length or native proteins. Immunogenic fragments such as truncated proteins are also contemplated, as are fragments which encode at least one HIV epitope, for example a CTL epitope, typically a peptide of at least 8 amino acids. Polynucleotides which encode a fragment of at least 8, for example 8-10 amino acids or up to 20, 50, 60, 70, 100, 150 or 200 amino acids in length are considered to fall within the scope of the invention as long as the encoded oligo or polypeptide demonstrates HIV antigenicity, that is to say that the major CTL epitopes are retained by the oligo or polypeptide. Major CTL epitopes are defined herein as those which are capable of eliciting an immune response in-vivo. The HIV polypeptide molecules encoded by the polynucleotide sequences according to the invention may represent a fragment of at least 50% of the length of the native protein, which fragment may contain mutations but which retains at least one HIV epitope and demonstrates HIV antigenicity. Such HIV antigenicity can be measured for example by measuring antibody or cell-mediated responses. Similarly, immunogenic derivatives disclosed must demonstrate HIV antigenicity. Immunogenic derivatives may provide some potential advantage over the native protein such as reduction or removal of a function of the native protein which is undesirable in a vaccine antigen such as enzyme activity (RT), or CD4 downregulation (Nef). The polynucleotide sequences may be codon optimised for mammalian cells, in line with codon optimization aspects of the invention as described herein.

The present invention further provides a method of preparing a vector according to the invention comprising the steps of:
a) providing an adenovirus vector;
b) providing a plasmid carrying the HIV antigen sequences operably linked to a suitable promoter;
c) transfecting cells with both the plasmid and the vector;
d) allowing sufficient time for recombination to occur; and
e) recovering recombinant virus vector carrying the HIV antigen sequences.

In another aspect, the present invention provides a method of raising an immune response in a mammal which method comprises administering to the mammal a suitable amount of an immunogenic composition according to the invention.

The invention may relate in particular to HIV-1. The constructs described herein may be derived from any HIV clade, for example clade B or clade C, particularly clade B.

A promoter for use in the adenovirus vector according to the invention may be the promoter from HCMV IE gene, for example wherein the 5' untranslated region of the HCMV IE gene comprising exon 1 is included as described in WO 02/36792.

The pharmaceutical composition can be administered in sufficient amounts to transduce the target cells and to provide sufficient levels of gene transfer and expression to provide a therapeutic benefit without undue adverse or with medically acceptable physiological effects, which can be determined by those skilled in the medical arts. Conventional and pharmaceutically acceptable routes of administration include, but are not limited to, direct delivery to the retina and other intraocular delivery methods, direct delivery to the liver, inhalation, intranasal, intravenous, intramuscular, intratracheal, subcutaneous, intradermal, rectal, oral and other parenteral routes of administration. Routes of administration may be combined, if desired, or adjusted depending upon the gene product or the condition. The route of administration primarily will depend on the nature of the condition being treated.

Dosages of the viral vector will depend primarily on factors such as the condition being treated, the age, weight and health of the patient, and may thus vary among patients. For example, a therapeutically effective adult human or veterinary dosage of the viral vector is generally in the range of from about 100 µL to about 100 mL of a carrier containing concentrations of from about 1 x 10⁶ to about 1 x 10¹⁵ particles, about 1 x 10¹¹ to 1 x 10¹³ particles, or about 1 x 10⁹ to 1x 10¹² particles virus. Dosages will range depending upon the size of the animal and the route of administration. For example, a suitable human or veterinary dosage (for about an 80 kg animal) for intramuscular injection is in the range of about 1 x 10⁹ to about 5 x 10¹² particles per mL, for a single site. Optionally, multiple sites of administration may be delivered. In another example, a suitable human or veterinary dosage may be in the range of about 1 x 10" to about 1 x 10¹⁵ particles for an oral formulation. One of skill in the art may adjust these doses, depending on the route of administration, and the therapeutic or vaccinal application for which the recombinant vector is employed. The levels of expression of the therapeutic product, or for an immunogen, the level of circulating antibody, can be monitored to determine the frequency of dosage administration. Yet other methods for determining the timing of frequency of administration will be readily apparent to one of skill in the art.

Administration of the pharmaceutical composition may take the form of one or of more than one individual dose, for example as repeat doses of the same polynucleotide containing adenovirus, or in a heterologous "prime-boost" vaccination regime. A heterologous prime-boost regime uses administration of different forms of vaccine in the prime and the boost, each of which may itself include two or more administrations. The priming composition and the boosting composition will have at least one antigen in common, although it is not necessarily an identical form of the antigen, it may be a different form of the same antigen.

A prime boost regime of use with the vectors of the present invention may take the form of a heterologous DNA and adenoviral vector prime boost, for example, a naked DNA priming dose, followed by an adenoviral vector boost, or for example, an adenoviral vector prime followed by one or more naked DNA boosts. Such DNA boosts may be delivered by intra-muscular or intra-dermal administration of DNA, or by particle acceleration techniques. Alternatively such a prime boost regime could comprise for example a protein and adenoviral vector according to the present invention, with the priming dose comprising the protein, and the boosting dose comprising the adenoviral vector or for example wherein the priming dose comprises an adenoviral vector and the boosting dose comprises a protein.

### Examples

### Example 1.

### Construction of the E1/E3 deleted Pan 6 and 7 Adenovirus

### 1. Generation of Recombinant E1-Deleted SV-25 Vector

A plasmid was constructed containing the complete SV-25 genome except for an engineered E1 deletion. At the site of the E1 deletion recognition sites for the restriction enzymes I-CeuI and PI-SceI which would allow insertion of transgene from a shuttle plasmid where the transgene expression cassette is flanked by these two enzyme recognition sites were inserted.

A synthetic linker containing the restriction sites SwaI-SnaBI-SpeI-AflII-EcoRV-SwaI was cloned into pBR322 that was cut with EcoRI and Ndel. This was done by annealing together two synthetic oligomers SV25T (5'-AAT TTA AAT ACG TAG CGC ACT AGT CGC GCT AAG CGC GGA TAT CAT TTA AA-3') and SV25B (5'-TAT TTA AAT GAT ATC CGC GCT TAA GCG CGA CTA GTG CGC TAC GTA TTT A-3') and inserting it into pBR322 digested with EcoRI and Ndel. The left end (bp1 to 1057) of Ad SV25 was cloned into the above linker between the SnaBI and Spel sites. The right end (bp28059 to 31042) of Ad SV25 was cloned into the linker between the AflII and EcoRV sites. The adenovirus E1 was then excised between the EcoRI site (bp 547) to XhoI (bp 2031) from the cloned left end as follows. A PCR generated I-CeuI-PI-SceI cassette from pShuttle (Clontech) was inserted between the EcoRI and Spel sites. The 10154 bp Xhol fragment of Ad SV-25 (bp2031 to 12185) was then inserted into the Spel site. The resulting plasmid was digested with HindIII and the construct (pSV25) was completed by inserting the 18344 bp Ad SV-25 HindIII fragment (bp11984 to 30328) to generate a complete molecular clone of E1 deleted adenovirus SV25 suitable for the generation of recombinant adenoviruses. Optionally, a desired transgene is inserted into the I-CeuI and PI-SceI sites of the newly created pSV25 vector plasmid.

To generate an AdSV25 carrying a marker gene, a GFP (green fluorescent protein) expression cassette previously cloned in the plasmid pShuttle (Clontech) was excised with the restriction enzymes I-Ceul and PI-Scel and ligated into pSV25 (or another of the Ad chimp plasmids described herein) digested with the same enzymes. The resulting plasmid (pSV25GFP) was digested with Swal to separate the bacterial plasmid backbone and transfected into the E1 complementing cell line HEK 293. About 10 days later, a cytopathic effect was observed indicating the presence of replicative virus. The successful generation of an Ad SV25 based adenoviral vector expressing GFP was confirmed by applying the supernatant from the transfected culture on to fresh cell cultures. The presence of secondarily infected cells was determined by observation of green fluorescence in a population of the cells.

### 2. Construction of E3 deleted Pan-6 and Pan-7 vectors.

In order to enhance the cloning capacity of the adenoviral vectors, the E3 region can be deleted because this region encodes genes that are not required for the propagation of the virus in culture. Towards this end, E3-deleted versions of Pan-5, Pan-6, Pan-7, and C68 have been made (a 3.5 kb Nru-AvrII fragment containing E31-9 is deleted).

### E3 deletion in Pan6 based vector

E1-deleted pPan6- pkGFP molecular clone was digested with Sbf I and Not I to isolate 19.3 kb fragment and ligated back at Sbf I site. The resulting construct pPan6-Sbf I-E3 was treated with Eco 47 III and Swa I, generating pPan6-E3. Finally, 21 kb Sbf I fragment from Sbf I digestion of pPan6- pkGFP was subcloned into pPan6-E3 to create pPan6-E3-pkGFP with a 4 kb deletion in E3.

### E3 deleted Pan7 vector

The same strategy was used to achieve E3 deletions in Pan 7. First, a 5.8 kb Avr II fragment spanning the E3 region was subcloned pSL-1180, followed by deletion of E3 by Nru I digestion. The resulting plasmids were treated with Spe I and Avr II to obtain 4.4 kb fragments and clone into pPan7- pkGFP at Avr II sites to replace the original E3 containing Avr II fragments, respectively. The final pPan7-E3- pkGFP construct had a 3.5 kb E3-deletion.

A full description of construction of E1, E3 and E4 deletions in these and other Pan Adenovirus serotypes is given in WO03/0046124. Further information is also available in Human Gene Therapy 15:519-530 (WO03/046124).

### Example 2.

### Construction of Gag, RT, Nef sequence.

This is described in full in WO03/025003

### Plasmid p73i-Tgrn

### 1. Plasmid: p73i-GRN2 Clone #19 (p17/p24(opt)/RT(opt)trNef) - repaired

### Gene of interest:

The p17/p24 portion of the codon optimised Gag, codon optimised RT and truncated Nef gene from the HIV-1 clade B strain HXB2 downstream of an Iowa length HCMV promoter + exon1, and upstream of a rabbit β-globin poly-adenylation signal.

Plasmids containing the trNef gene derived from plasmid p17/24trNef1 contain a PCR error that gives an R to H amino acid change 19 amino acids from the end of Nef. This was corrected by PCR mutagenesis, the corrected Nef PCR stitched to codon optimised RT from p7077-RT3, and the stitched fragment cut with ApaI and BamHI, and cloned into ApaI/BamHI cut p73i-GRN.

### Primers:

PCR coRT from p7077-RT3 using primers:
(Polymerase = PWO (Roche) throughout.

### Sense: U1

GAATTCGCGGCCGCGATGGGCCCCATCAGTCCCATCGAGACCGTGCCGGTGA
AGCTGAAACCCGGGAT

AScoRT-Nef
GGTGTGACTGGAAAACCCACCATCAGCACCTTTCTAATCCCCGC

Cycle: 95°C(30s) then 20 cycles 95°C(30s), 55°C(30s), 72°C(180s), then 72°C(120s) and hold at 4°C
The 1.7kb PCR product was gel purified.

PCR 5' Nef from p17/24trNef1 using primers:
Sense: S-Nef
ATGGTGGGTTTTCCAGTCACACC
Antisense: ASNef-G:
GATGAAATGCTAGGCGGCTGTCAAACCTC
Cycle: 95°C(30s) then 15 cycles 95°C(30s), 55°C(30s), 72°C(60s), then 72°C(120s) and hold at 4°C

PCR 3' Nef from p17/24trNef1 using primers:
Sense: SNEF-G
GAGGTTTGACAGCCGCCTAGCATTTCATC
Antisense:
AStrNef (antisense)
CGCGGATCCTCAGCAGTTCTTGAAGTACTCC
Cycle: 95°C(30s) then 15 cycles 95°C(30s), 55°C(30s), 72°C(60s), then 72°C(120s) and hold at 4°C

The PCR products were gel purified. Initially the two Nef products were stitched using the 5' (S-Nef) and 3' (AstrNef) primers.
Cycle: 95°C(30s) then 15 cycles 95°C(30s), 55°C(30s), 72°C(60s), then 72°C(180s) and hold at 4°C.
The PCR product was PCR cleaned, and stitched to the RT product using the U1 and AstrNef primers:
Cycle: 95°C(30s) then 20 cycles 95°C(30s), 55°C(30s), 72°C(180s), then 72°C(180s) and hold at 4°C

The 2.1 kb product was gel purified, and cut with Apal and BamHI. The plasmid p73I-GRN was also cut with Apa1 and BamHI gel purified and ligated with the Apal-Bam RT3trNef to regenerate the p17/p24(opt)/RT(opt)trNef gene.

### 2. Plasmid: p731-RT w229k (Inactivated RT)

### Gene of Interest:

Generation of an inactivated RT gene downstream of an Iowa length HCMV promoter + exon 1, and upstream of a rabbit β-globin poly-adenylation signal.

Due to concerns over the use of an active HIV RT species in a therapeutic vaccine inactivation of the gene was desirable. This was achieved by PCR mutagenesis of the RT (derived from P73I-GRN2) amino acid position 229 from Trp to Lys (R7271 p1-28).

### Primers:

PCR 5' RT + mutation using primers:
(polymerase = PWO (Roche) throughout)

Sense : RT3-u:1

GAATTCGCGGCCGCGATGGGCCCCATCAGTCCCATCGAGACCGTGCCGGTGA
AGCTGAAACCCGGGAT

Antisense: AScoRT-Trp229Lys
GGAGCTCGTAGCCCATCTTCAGGAATGGCGGCTCCTTCT

Cycle:
1 x [94°C (30s)]
15 x [94°C (30s)/55°C (30s)/72°C (60s)]
1 x [72°C (180s)]
PCR gel purify

PCR 3' RT + mutation using primers:

Antiense: RT3- I-1

GAATTCGGATCCTTACAGCACCTTTCTAATCCCCGCACTCACCAGCTTGTCGAC
CTGCTCGTTGCCGC

Sense: ScoRT-Trp229Lys
CCTGAAGATGGGCTACGAGCTCCATG

Cycle:
1 x [94°C (30s)]
15 x [94°C (30s)/55°C (30s)/72°C (60s)]
1 x [72°C (180s)]
PCR gel purify

The PCR products were gel purified and the 5' and 3' ends of RT were stitched using the 5' (RT3-U1) and 3' (RT3-L1) primers.
Cycle:
1 x [94°C (30s)]
15 x [94°C (30s)/55°C (30s)/72°C (120s)]
1 x [72°C (180s)]

The PCR product was gel purified, and cloned into p7313ie, utilising Notl and BamHI restriction sites, to generate p731-RT w229k. (See figure 13)

### 3. Plasmid: p731-Tgrn

### Gene of interest:

The p17/p24 portion of the codon optimised gag, codon optimised RT and truncated Nef gene from the HIV-1 clade B strain HXB2 downstream of an Iowa length HCMV promoter + exon1, and upstream of a rabbit β-globin poly-adenylation signal.

Triple fusion constructs which contain an active form of RT, may not be acceptable to regulatory authorities for human use thus inactivation of RT was achieved by Insertion of a Nhel and Apal cut fragment from p73i-RT w229k, into Nhel/Apal cut p73i-GRN2#19 (Figure 14). This results in a W → K change at position 229 in RT.

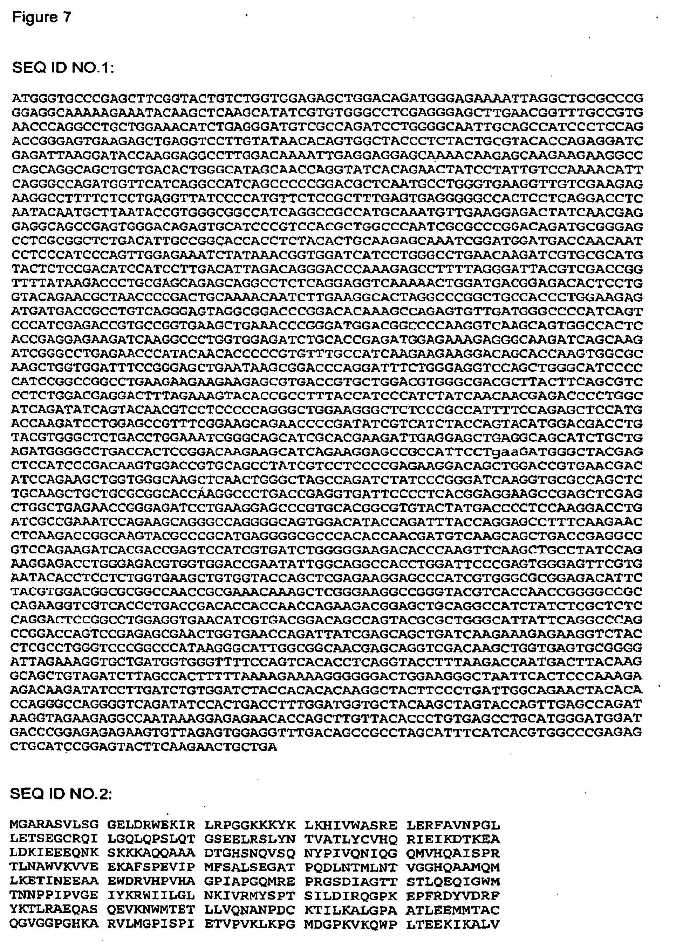

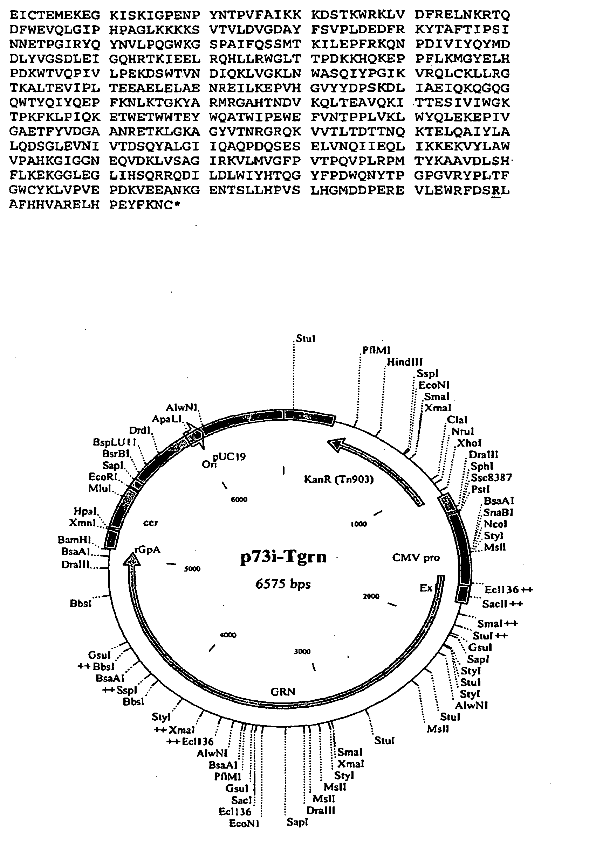

The full sequence of the Tgrn plasmid insert is shown in Fig 7. This contains p17 p24 (opt) Gag, p66 RT (opt and inactivated) and truncated Nef.

Alternative constructs of Gag, RT and Nef are as follows:
trNef - p66 RT (opt) - p17, p24 (opt) Gag,
trNef - p17, p24 (opt) Gag - p66 RT (opt),
p66 RT (opt) - p17, p24 (opt) Gag - trNef,
p66 RT (opt) - trNef - p17, p24 (opt) Gag.
p17, p24 (opt) Gag - trNef - p66 RT (opt).

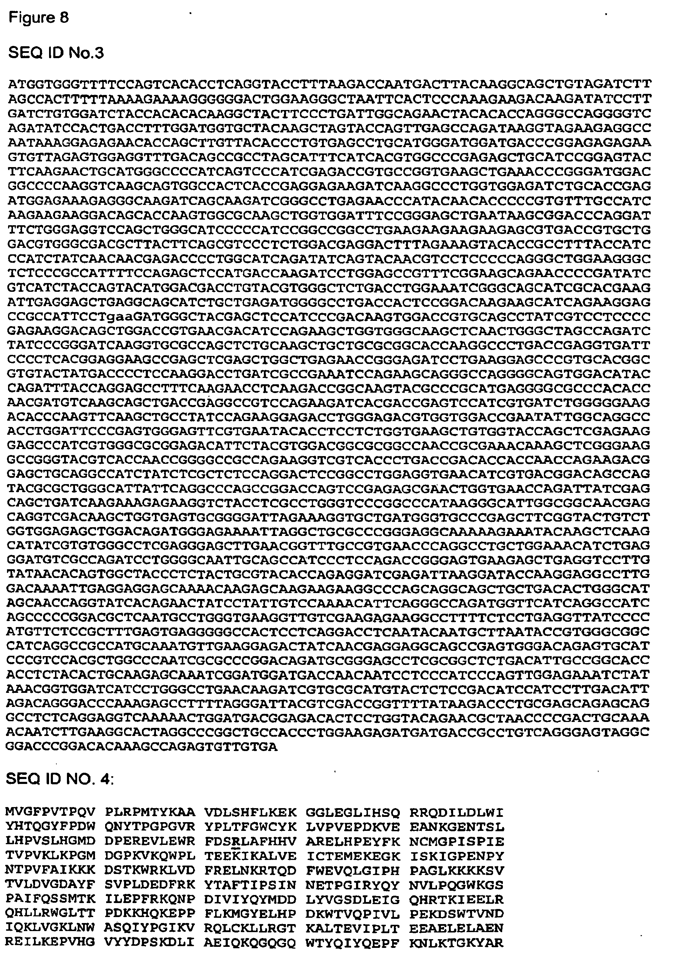

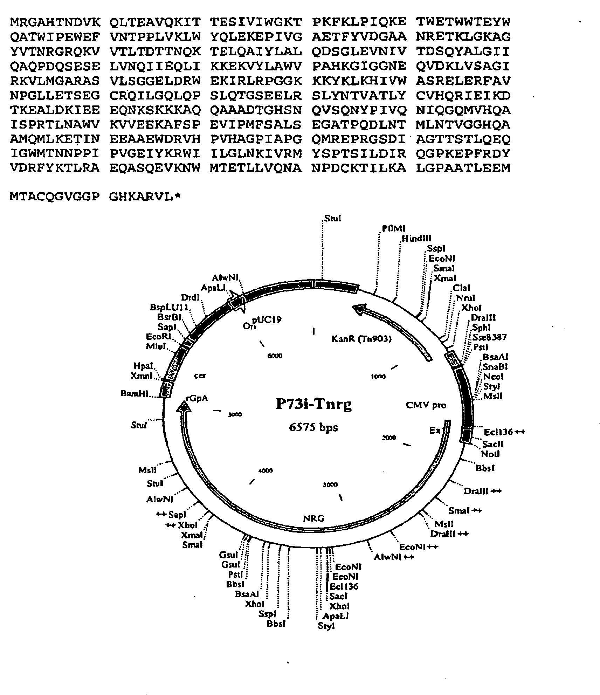

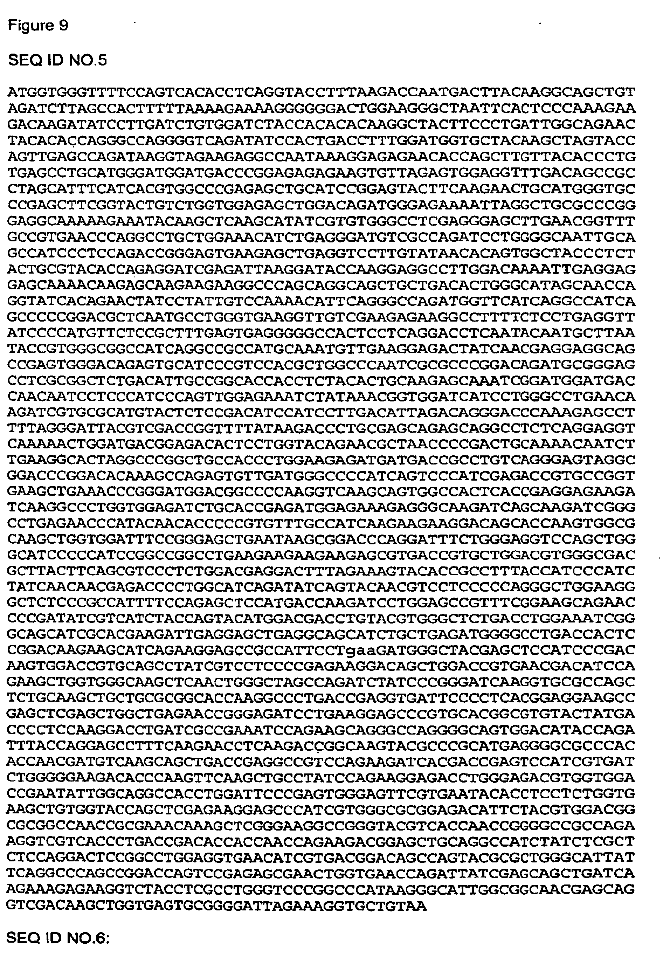

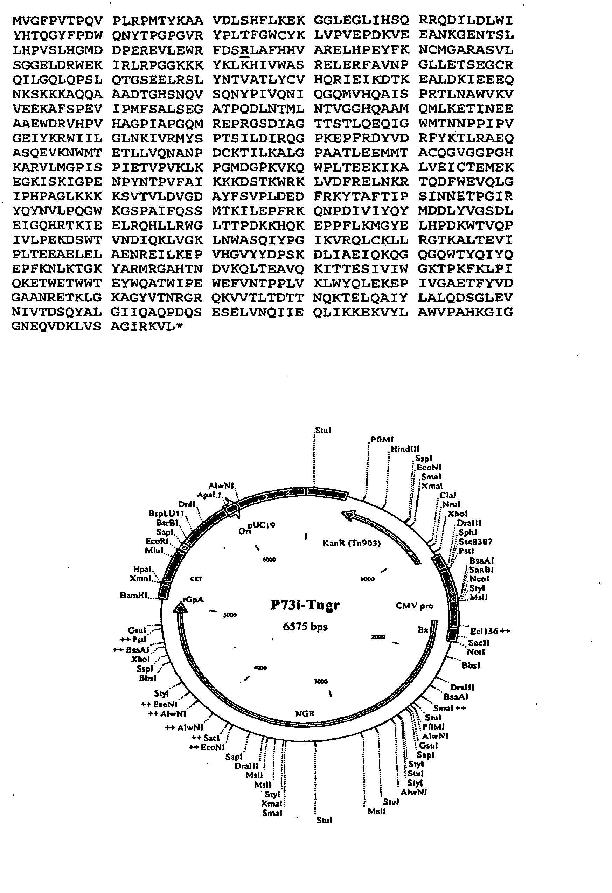

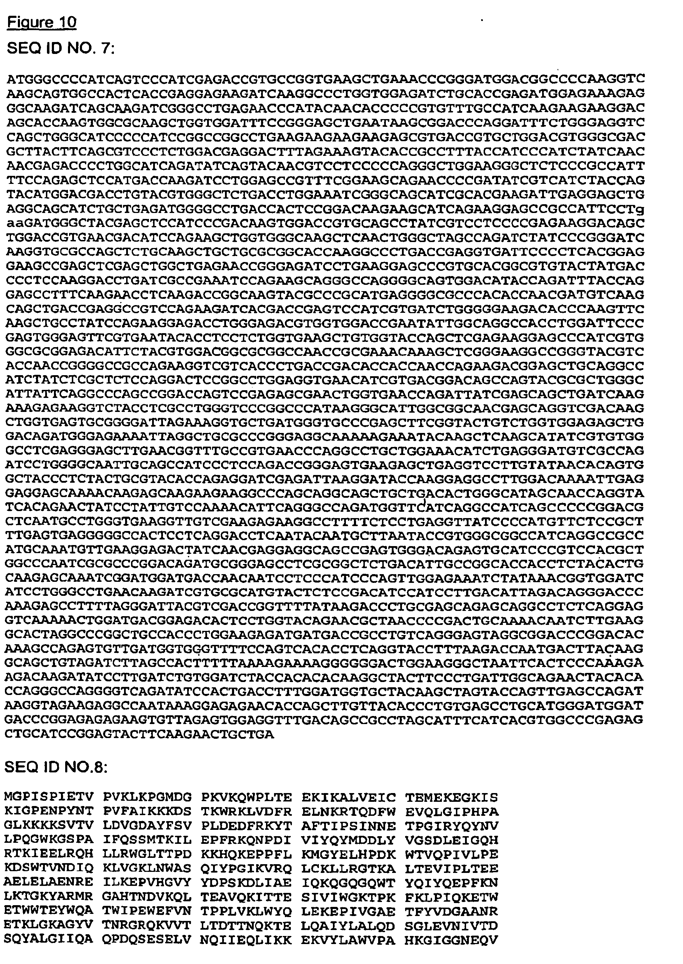

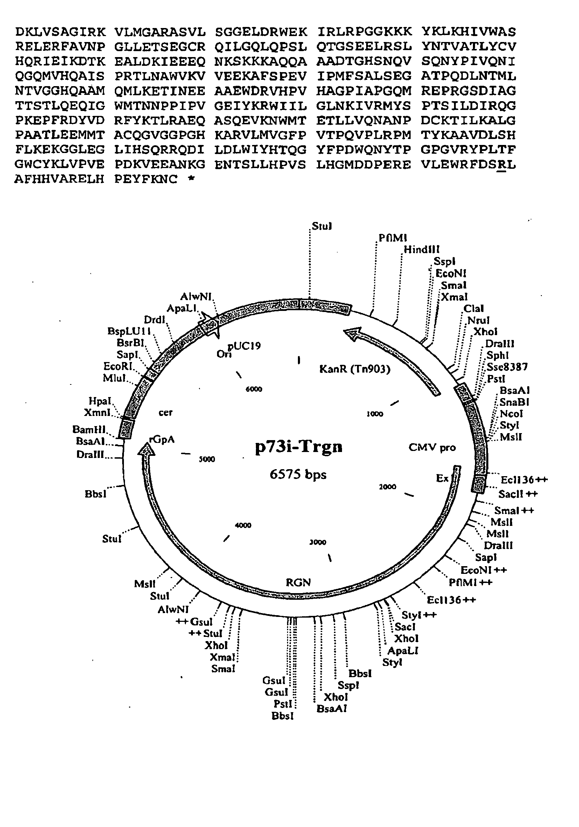

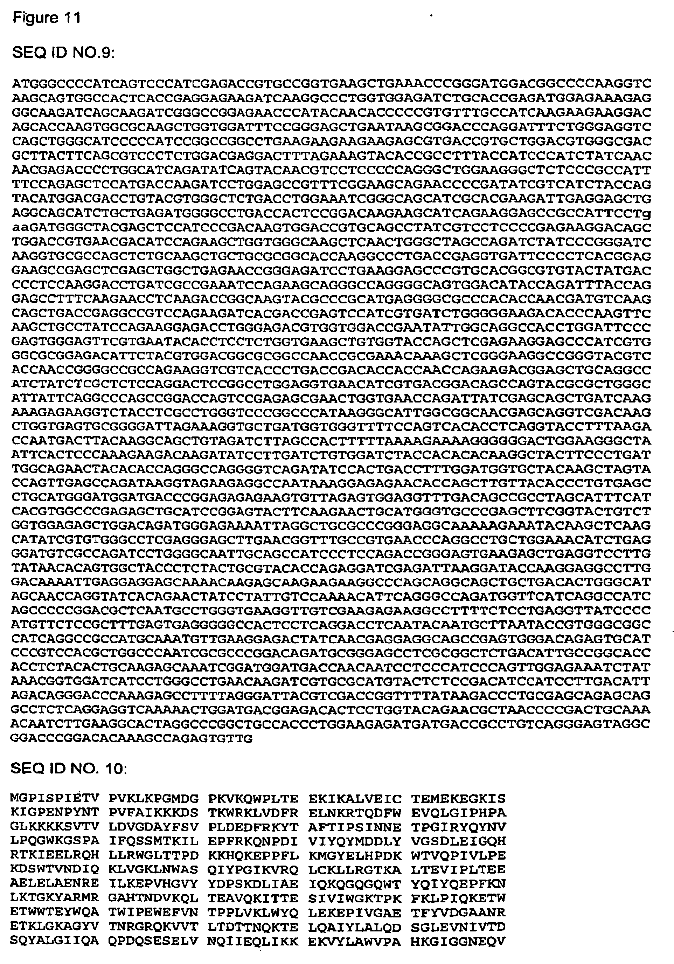

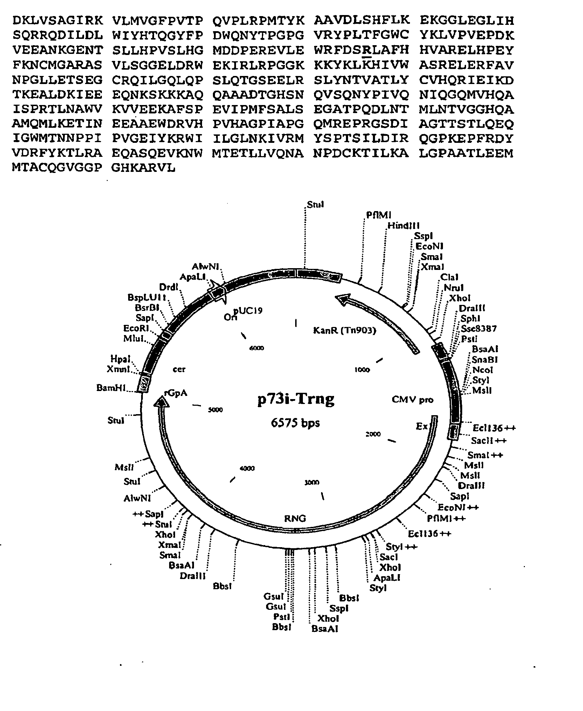

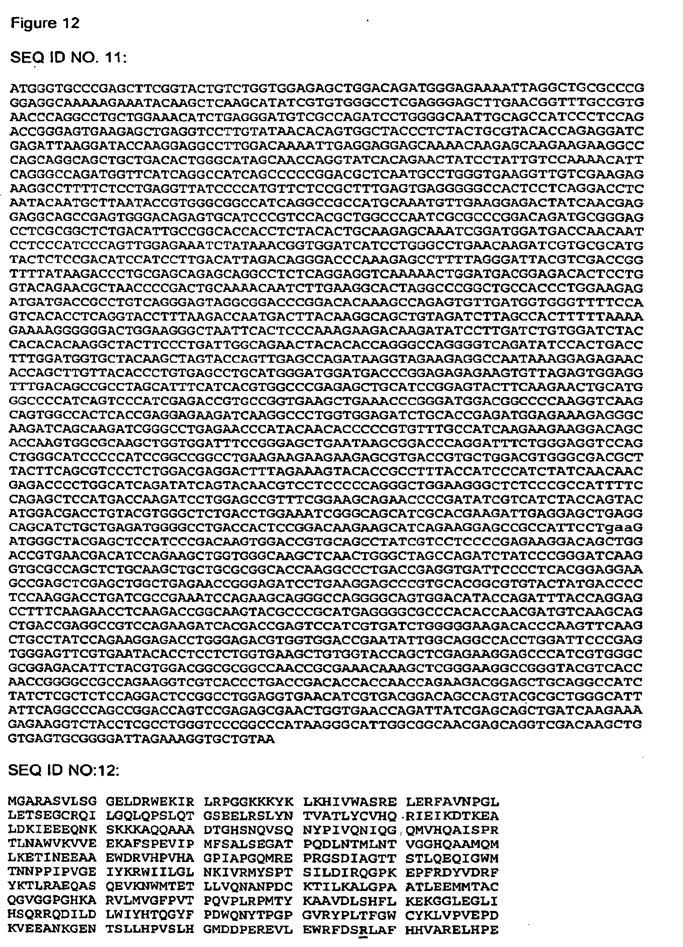

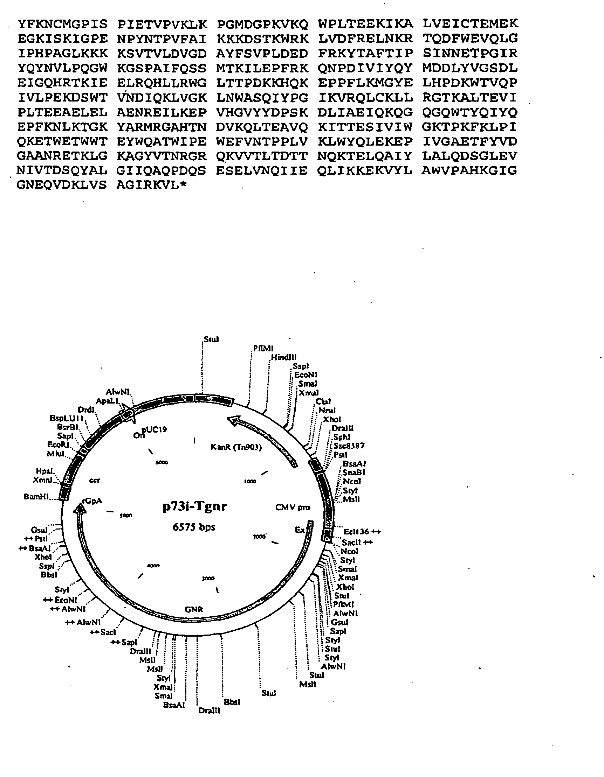

Full sequences for these constructs are given in Figures 8 to 12 respectively.

### Example 3.

### Insertion of Gag, RT, Nef sequence into Adenovirus.

### Subcloning of GRN expression cassette into pShuttle plasmid.

The entire expression cassette consisting of promoter, cDNA and polyadenylation signal was isolated from pT-GRN constructs by Sph I and EcoR I double digestion. The Sph I end of the Sph I/EcoR I fragment was filled in with Klenow and cloned into pShuttle plasmid at EcoR I and Mlu I sites where the Mlu I end was blunted.

During the cloning process an additional flanking sequence became associated with the HIV expression cassette. This sequence is known as the Cer sequence and has no known function.

### Transfer of GRN expression cassette into E1/E3-deleted molecular clones of Pan6 and Pan7 vectors.

The expression cassette was retrieved from pShuttle by I-Ceu I and PI-Sce I digestions and cloned into the same sites of the molecular clones of Pan6 and Pan7 vectors. Recombinant clones were identified through green/white selection and confirmed by extensive restriction enzyme analysis.

### Rescue and propagation of recombinant viruses.

Molecular clones of C6 and C7 vectors were treated with appropriate restriction endonucleases (Pmel and Pacl respectively) to release intact linear vector genomes and transfected into 293 cells using the calcium phosphate method. When full cytopathetic effect was observed in the transfected cells, crude viral lysate was harvested and gradually expanded to large scale infections in 293 cells (1x10e9 cells). Viruses from large scale infections were purified by standard CsCl sedimentation method.

In addition the pShuttle plasmid can be further trimmed by cutting with EcoRI and Xmnl to remove a 3' linker sequence and reduce the plasmid size to produce pShuttleGRNc. This modified plasmid can be used to generate an additional Pan7 virus (C7-GRNc) using the method as described above.

Other constructs were similarly inserted into both the Pan 6 and Pan 7 adenovirus. However Pan 6 with a p66 RT (opt) - trNef - p17, p24 (opt) Gag insert was not successfully produced.

### Example 4.

### Mouse Immunogenicity model

A series of Pan6 and Pan7 vectors containing rearranged inserts of the HIV antigens RT, Nef and Gag (RGN, NRG, NGR, GRN, and GNR) were tested for primary immune responses *in vivo.* Three experiments were conducted to test the Pan6 viruses and two for Pan7. Each adenovirus was administered intra-muscularly in a 50µl volume to a single hind limb of Balb/c (K2^{d}) mice at a dose of 1x10⁸ particles. This dose was selected as it had previously been shown to induce good levels of cellular immune responses (unpublished).
Table 1 outlines the adenoviruses that were compared in these experiments.

**Table 1**

| **Group** | **Immunisation Pan6** | **Immunisation Pan7** |
|---|---|---|
| | **Week 0** | **Week 0** |
| 1 | Pan6-NRG 10⁸ | Pan7-NRG 10⁸ |
| 2 | Pan6-NGR 10⁸ | Pan7-NGR 10⁸ |
| 3 | Pan6-RGN 10⁸ | Pan7-RNG 10⁸ |
| 4 | Pan6-GRN 10⁸ | Pan7-RGN 10⁸ |
| 5 | Pan6-GNR 10⁸ | Pan7-GRN 10⁸ |
| 6 | DNA: P7313 | Pan7-GNR 10⁸ |
| 7 | | DNA: P7313 |

Following *in vitro* stimulation with peptides or proteins to specific epitopes in Gag, Nef and RT the generation of CD8 and CD4 responses were measured by ELIspot assay at 14 and 28 days post prime. The results provide strong evidence that all the variants are able to generate a potent primary immune response as measured by the production of both IFN γ and IL-2 compared with the empty vector control (data not shown).

The data from these studies was statistically analysed (using a mixed model analysis of variance (ANOVA) in Proc Mixed in SAS (version 9.1.3 Service Pack 2) to determine a ranking of the RNG variants in Pan6 and Pan7 at separate time points. The sum of responses to the CD8 peptides for IFN γ production were quantified for Gag and RT whereas the IL-2 ELIspot data were evaluated on the sum of responses to the CD4 peptides for Gag, Nef and RT.

The ranking of the panel of variants was calculated using the Bayesian model (performed using the Prior statement in Proc Mixed with a flat prior generating 100,000 posterior samples; see Tierney, L. (1994), "Markov Chains for Exploring Posterior Distributions" (with discussion), and Annals of Statistics, 22, 1701 - 1762. Gelfand, A.E., Hills, S.E., Racine-Poon, A., and Smith, A.F.M. (1990), "Illustration of Bayesian Inference in Normal Data Models Using Gibbs Sampling," Journal of the American Statistical Association, 85, 972 - 985) to forecast the probability of each of the variants as the 'best', based on the data provided by the experimental conditions investigated.

Figure 1 represents the sum of the Pan6 CD4 and CD8 responses for IFN γ and IL-2 with each peptide at day 14 and 28 as predicted by the Bayesian method.

Figure 2 represents the sum of the Pan7 CD4 and CD8 responses for IFN γ and IL-2 with each peptide at day 14 and 28 as predicted by the Bayesian method.

All the inserts show a significant increase in immune responses compared with the empty vector control. The statistical analysis shows that there are no significant differences between the different viruses.

### Example 5.

### Pig Immunogenicity model

Results from several studies have indicated that the pig is a good model for testing immunogenicity of candidate vaccines. A study was set up to investigate the immunogenicity of the four candidate NHP adenoviruses in minipigs. Groups of 5 minipigs were primed with PAN6GRN, PAN6NGR, PAN7GRN or PAN7NGR (for details of batches used see Table 2). Each animal received a total of 3 x 10¹⁰ virus particles of adenovirus via the intramuscular route (using a 1.0 ml volume divided equally between each medial thigh muscle).

**Table 2. Batches of NHP adenoviruses used for the minipig experiment**

| **Group** | **Vector** | |
|---|---|---|
| | Week 0 | Week 12 |
| 1 | PAN6GRN | PAN6GRN |
| 2 | PAN6NGR | PAN6NGR |
| 3 | PAN7GRN | PAN7GRN |
| 4 | PAN7NGR | PAN7NGR |
| 5 | PAN6NGR | PAN6NGR |

Blood samples were collected before immunisation and at intervals post-immunisation from every animal. The peripheral blood mononuclear cells were isolated and restimulated in vitro with RT, Nef and Gag peptide library pools and proteins. The peptide library pools consist of 15-mer peptides overlapping by 11 amino acids spanning the entire sequence of RT, Nef and Gag and were the same as those used for the in vivo mouse experiments.

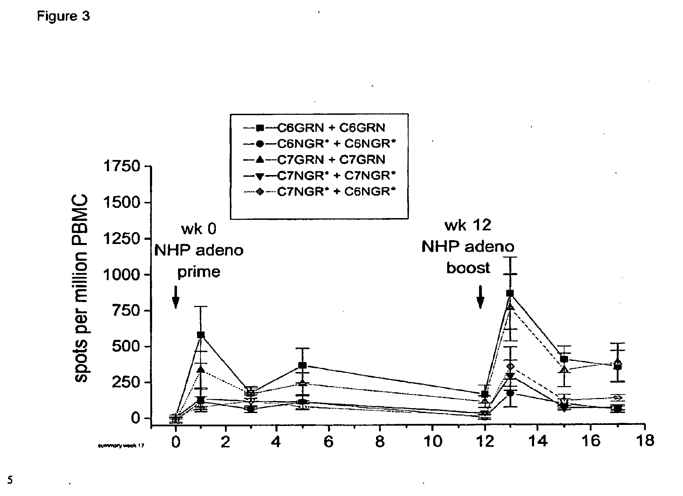

The production of interferon-gamma by these porcine cells has been measured using ELIspot assays. Figure 3 shows the responses to RT, Nef and Gag peptide library pools at the 4 sampling time points.

Responses are detected to all four viruses seven days post immunisation. Cellular response to all four NHP viruses is maintained until at least 5 weeks post-primary. PAN6-GRN generates the strongest response at 7 days post-primary by IFN-gamma ELIspot.

### Example 6.

### Primate Immunogenicity Model

Results from a primate pilot study indicated that intramuscular injection of NHP adenoviruses expressing RT, Nef and Gag elicited cellular immune responses in cynomolgus monkeys.

A study was set up to investigate the immunogenicity of the four candidate NHP adenoviruses in cynomolgus monkeys. Groups of animals were primed with PAN6-GRN, PAN6-NGR, PAN7-GRN or PAN7-NGR (for details of virus batches used see Table 3). Each animal received a total of 10" virus particles of adenovirus via the intramuscular route (using a 1.0 ml volume divided equally between each medial thigh muscle).

**Table 3. Batches of NHP adenoviruses used for the primate experiment**

| **Group** | **Immunisation Week 0** | **Animal i/d** |
|---|---|---|
| 1 | PAN6GRN | 18173, 18180, 18240, 18217, 18221 |
| 2 | PAN6NGR | 18144, 18155, 18199, 18216, 18238 |
| 3 | PAN7GRN | 18156, 8188, 18192, 18215, 18237 |
| 4 | PAN7NGR | 18160, 18170, 18208, 18226, 18236 |
| 5 | PAN7NGR | 18165, 18168, 18189, 18234 |

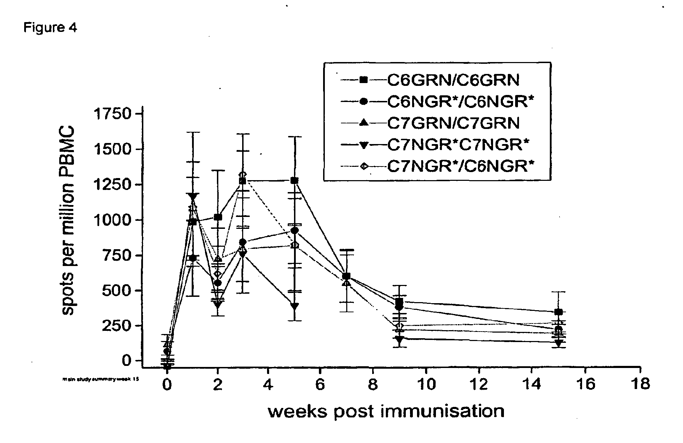

Blood samples were collected before immunisation and at weekly intervals thereafter. Peripheral blood mononuclear cells were isolated and restimulated in vitro with RT, Nef and Gag peptide library pools. The production of interferon-gamma by these primate cells was measured using ELIspot assays. Figure 4 shows the response of each group at the three sampling time points.

The results show that all groups responded strongly at one week after the primary immunisation, with responses maintained until at least 7 weeks post immunisation. The results suggest that there is little difference between the vectors when used at this dose (ie. 10¹¹ particles) in primates.

### Example 7.

Post-primary immune responses to a dose range of NHP Adenovirus encoding HIV GRN antigens delivered intra muscularly (i.m.).

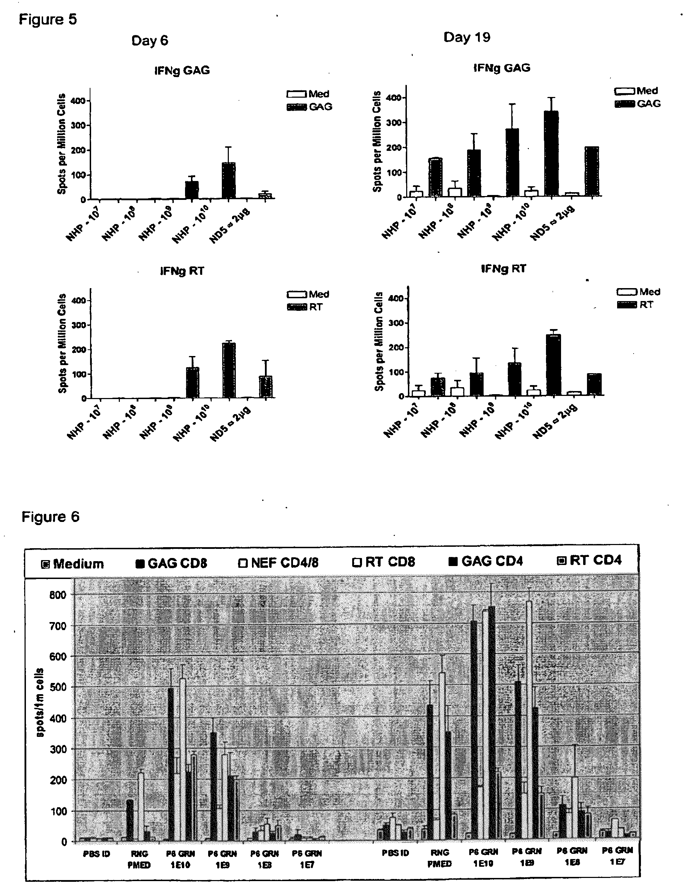

To evaluate the impact of the dose of adenovirus in primary immunization, a group of mice (n=5) were immunised intra muscularly (i.m.) with increasing doses of NHP Adenovirus (from 10⁷ to 10¹⁰ particles). As positive control a group of animals was immunised by DNA (2µg) using particle mediated epidermal delivery (ND5). On day 6 and day 19 post immunisation the animals were schedule one and spleen removed. Immune responses were monitored by IFN-γ ELISPOT assay using a peptide library pool for each of the antigens (GAG and RT) to stimulate the splenocytes overnight. Figure 5 shows the responses of each group at the two sampling time points.

### Example 8

Post-primary immune responses to a dose range of NHP Adenovirus encoding HIV GRN antigens delivered intra dermally (i.d.).

To evaluate the impact of the dose of adenovirus in primary immunization, a group of mice (n=5) were immunised intra dermally (i.d.) with increasing doses of NHP Adenovirus (from 10⁷ to 10¹⁰ particles). As positive control a group of animals was immunised by DNA (1µg) using particle mediated epidermal delivery (PMED). On day 7 and day 14 post immunisation the animals were schedule one and spleen removed. Immune responses were monitored by IFN-γ ELISPOT assay. Splenocytes were stimulated overnight using well defined peptides for each antigens (GAG and RT) that stimulate specifically CD4 or CD8 T-cells. Figure 6 shows the responses of each group at the two sampling time points.

These results suggest that both i-m and i-d are effective routes of administration of compositions of the invention.

### Description of Figures

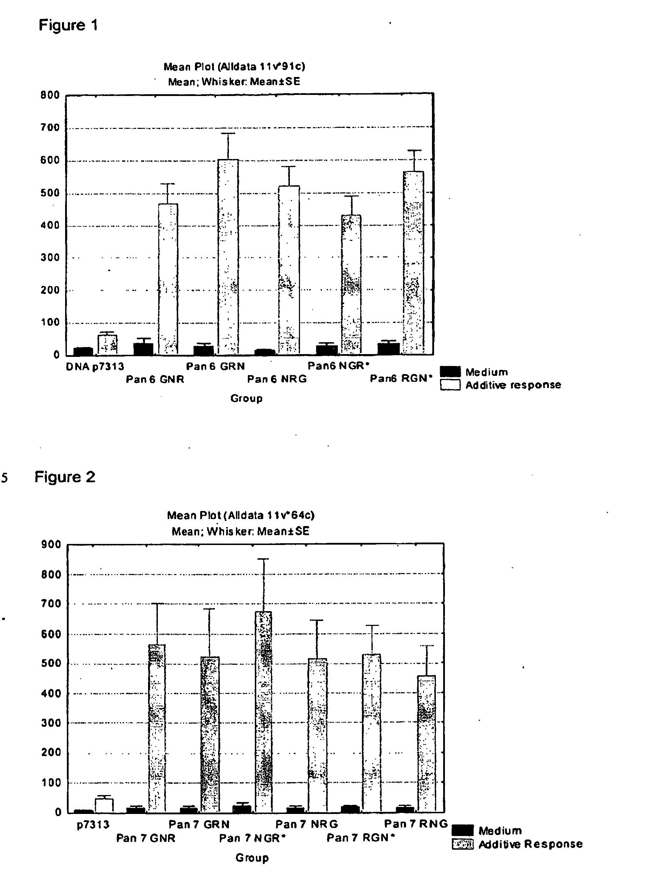

Figure 1. Ranking of Pan6 HIV Adenoviruses. This represents the sum of the Pan6 CD4 and CD8 responses for IFN γ and IL-2 with each peptide at day 14 and 28 as predicted by the Bayesian method. The y-axis represents spot forming cells per million splenoctyes.
Figure 2. Ranking of Pan7 HIV Adenoviruses. This represents the sum of the Pan7 CD4 and CD8 responses for IFN γ and IL-2 with each peptide at day 14 and 28 as predicted by the Bayesian method. The y-axis represents spot forming cells per million splenoctyes.
Figure 3. Responses of minipigs to RT, Nef and Gag peptide library pools at 0, 1, 3 and 5 weeks post-primary immunisation. Results are the mean ± standard error of the sum of responses to each peptide library pool for each animal. Data obtained from the University of Pennsylvania.
Figure 4. Responses of primates to RT, Nef and Gag peptide library pools at 0. 1 and 2 weeks post-primary immunisation. Results are the mean ± standard error of the sum of responses to each peptide library pool for each animal.
Figure 5: Post-primary immune responses to a dose range of NHP Adenovirus encoding HIV GRN antigens delivered intra muscularly (i.m.). Group of mice (n=5) have been immunised with various doses of NHP Adenovirus (from 10⁷ to 10¹⁰ particles) and cellular immune responses against a peptide library pool for each antigen are monitored (day 6 and day 19) using IFN-γ ELISPOT assay.
Figure 6: Post-primary immune responses to a dose range of NHP Adenovirus encoding HIV GRN antigens delivered intra dermally (i.d.). Group of mice (n=3) have been immunised with various doses of NHP Adenovirus (from 10⁷ to 10¹⁰ particles) and cellular immune responses against specific peptides are monitored (day7 and day 14) using IFN-γ ELISPOT assay.
Figures 7 to 12: Polynucleotide sequences, amino acid sequences and restriction maps for constructs described in Example 2.

Details of the sequences are set out in Table 4

**Table 4:**

| Amino acid or polynucleotide description | Sequence Identifier (SEQ ID No) |
|---|---|
| Tgrn polynucleotide | 1 |
| Tgrn amino acid | 2 |
| Tnrg polynucleotide | 3 |
| Tnrg amino acid | 4 |
| Tngr polynucleotide | 5 |
| Tngr amino acid | 6 |
| Trgn polynucleotide | 7 |
| Trgn amino acid | 8 |
| Trng polynucleotide | 9 |
| Trng amino acid | 10 |
| Tgnr polynucleotide | 11 |
| Tgnr amino acid | 12 |

## Claims

1. An adenovirus vector comprising a polynucleotide or polynucleotides encoding at least HIV antigens RT, Nef and Gag or immunogenic fragments thereof arranged so that they are transcribed in the order Gag, RT, Nef, so that the Gag portion is at the N terminal end of the resulting fusion protein, **characterized in that** the virus is a non-human primate adenovirus, and the virus is a chimpanzee adenovirus selected from Pan 5, Pan 6 and Pan 7.

2. The adenovirus according to claim 1 wherein the RT is truncated.

3. The adenovirus according to claim 1 or 2 wherein the Nef is truncated.

4. The adenovirus according to any one of claims 1 to 3 wherein the Gag is p17 and p24 only.

5. The adenovirus vector according to any preceding claim wherein the size of the HIV polynucleotide or polynucleotides is such that the overall size of the vector is from 90 to 100% of the size of the virus.

6. The adenovirus vector according to claim 5 wherein the adenovirus is pan 6.

7. The adenovirus vector according to claim 5 wherein the adenovirus is pan 7.

8. The adenovirus vector according to any preceding claim wherein the virus is replication defective.

9. The adenovirus vector according to any preceding claim wherein the virus is deleted in E1 and E3 regions.

10. The adenovirus vector according to any preceding claim wherein the polynucleotide sequences encoding the HIV antigens are arranged as a fusion.

11. The adenovirus vector according to claim 1 comprising the following polynucleotide constructs: p17, p24 (codon optimised) Gag- p66 RT (codon optimised)- truncated Nef.

12. An adenovirus vector according to claim 11 wherein the Adenovirus is Pan 6 or Pan 7.

13. An immunogenic composition comprising the virus vector according to any preceding claim and a pharmaceutically acceptable carrier or adjuvant.

14. The use of an adenovirus vector according to any one of claims 1 to 13 in the manufacture of a medicament for the treatment or prophylaxis of HIV infection.

15. A method of preparing a vector according to any one of the claims 1 to 13 comprising the steps of: a) providing an adenovirus vector; b) providing a plasmid carrying the HIV antigen sequences operably linked to a suitable promoter; c) transfecting cells with both the plasmid and the vector; d) allowing sufficient time for recombination to occur; and e) recovering recombinant virus vector carrying the HIV antigen sequences.

16. A method of raising an immune response in a mammal which method comprises administering to the mammal a suitable amount of an immunogenic composition according to claim 13.

17. A fusion protein expressed by the vector according to any one of claims 1to 12.

18. A fusion protein according to claim 17 produced within the human body.

## Patentansprüche

1. Adenovirus-Vektor, umfassend ein Polynucleotid oder Polynucleotide, die zumindest die HIV-Antigene RT, Nef und Gag codieren oder die immunogenen Fragmente davon, die so angeordnet sind, dass sie in der Reihenfolge Gag, RT, Nef transkribiert werden, so dass der Gag-Teil am N-terminalen Ende des resultierenden Fusionsproteins ist, **dadurch gekennzeichnet, dass** das Virus ein nicht-humanes Primaten-Adenovirus ist, und das Virus ein Schimpansen-Adenovirus ausgewählt aus Pan 5, Pan 6 und Pan7 ist.

2. Adenovirus nach Anspruch 1, wobei das RT trunkiert ist.

3. Adenovirus nach Anspruch 1 oder 2, wobei das Nef trunkiert ist.

4. Adenovirus nach einem der Ansprüche 1 bis 3, wobei das Gag nur p17 und p24 ist.

5. Adenovirus-Vektor nach einem der voranstehenden Ansprüche, wobei das HIV-Polypeptid oder die HIV-Polypeptide so groß ist/sind, dass die Gesamtgröße des Vektors von 90-100% der Größe des Virus hat.

6. Adenovirus-Vektor nach Anspruch 5, wobei das Adenovirus pan 6 ist.

7. Adenovirus-Vektor nach Anspruch 5, wobei das Adenovirus pan 7 ist.

8. Adenovirus-Vektor nach einem der voranstehenden Ansprüche, wobei das Virus Replikations-defekt ist.

9. Adenovirus-Vektor nach einem der voranstehenden Ansprüche, wobei das Virus in E1- und E3-Regionen deletiert ist.

10. Adenovirus-Vektor nach einem der voranstehenden Ansprüche, wobei die Polynucleotid-Sequenzen, die die HIV-Antigene codieren, als eine Fusion angeordnet sind.

11. Adenovirus-Vektor nach Anspruch 1, das die folgenden Polynucleotid-Konstrukte umfasst: p17, p24 (Codon-optimiertes) Gag- p66 RT (Codon-optimiertes)-trunkiertes Nef.

12. Adenovirus-Vektor nach Anspruch 11, wobei das Adenovirus Pan 6 oder Pan 7 ist.

13. Immunogene Zusammensetzung, umfassend den Virus-Vektor nach einem der voranstehenden Ansprüche und einen pharmazeutisch verträglichen Träger oder ein pharmazeutisch verträgliches Adjuvans.

14. Verwendung eines Adenovirus-Vektors nach einem der Ansprüche 1 bis 13 bei der Herstellung eines Medikaments für die Behandlung oder Vorbeugung von HIV-Infektionen.

15. Verfahren zur Herstellung eines Vektors nach einem der Ansprüche 1 bis 13, umfassend die Schritte: a) Bereitstellen eines Adenovirus-Vektors; b) Bereitstellen eines Plasmids, das die HIV-Antigensequenzen trägt, die mit einem geeigneten Promotor funktionell verknüpft sind; c) Transfektion von Zellen mit sowohl dem Plasmid als auch dem Vektor; d) Zugestehen von ausreichend Zeit, dass Rekombination stattfindet; und e) Gewinnen des rekombinanten Virus-Vektors, der die HIV-Antigensequenzen trägt.

16. Verfahren zum Hervorrufen einer Immunantwort in einem Säuger, wobei das Verfahren die Verabreichung einer geeigneten Menge einer immunogenen Zusammensetzung nach Anspruch 13 an den Säuger umfasst.

17. Fusionsprotein, das von einem Vektor nach einem der Ansprüche 1 bis 12 exprimiert wird.

18. Fusionsprotein nach Anspruch 17, das im menschlichen Körper produziert wird.

## Revendications

1. Vecteur adénoviral comprenant un ou plusieurs polynucléotides codant pour au moins les antigènes du VIH RT, Nef et Gag ou de leurs fragments immunogènes disposés de telle sorte qu'ils soient transcrits dans l'ordre Gag, RN, Nef, de telle sorte que la portion Gag soit à l'extrémité N-terminale de la protéine de fusion résultante, **caractérisé en ce que** le virus est un adénovirus de primate non humain, et le virus est un adénovirus de chimpanzé choisi entre Pan 5, Pan 6 et Pan 7.

2. Adénovirus suivant la revendication 1, dans lequel le RT est tronqué.

3. Adénovirus suivant la revendication 1 ou 2, dans lequel le Nef est tronqué.

4. Adénovirus suivant l'une quelconque des revendications 1 à 3, dans lequel le Gag est constitué de p17 et p24 seulement.

5. Vecteur adénoviral suivant l'une quelconque des revendications précédentes, dans lequel la dimension du ou des polynucléotides du VIH est telle que la dimension totale du vecteur représente 90 à 100 % de la dimension du virus.

6. Vecteur adénoviral suivant la revendication 5, dans lequel l'adénovirus est pan 6.

7. Vecteur adénoviral suivant la revendication 5, dans lequel l'adénovirus est pan 7.

8. Vecteur adénoviral suivant l'une quelconque des revendications précédentes, dans lequel le virus présente une réplication défective.

9. Vecteur adénoviral suivant l'une quelconque des revendications précédentes, dans lequel le virus présente une délétion dans les régions E1 et E3.

10. Vecteur adénoviral suivant l'une quelconque des revendications précédentes, dans lequel les séquences polynucléotidiques codant pour les antigènes du VIH sont disposées sous forme d'une fusion.

11. Vecteur adénoviral suivant la revendication 1, comprenant les produits d'assemblage polynucléotidiques suivants : p17, p24 (à optimisation des codons) Gag-p66 RT (à optimisation des codons)-Nef tronqué.

12. Vecteur adénoviral suivant la revendication 11, dans lequel l'adénovirus est Pan 6 ou Pan 7.

13. Composition immunogène comprenant le vecteur viral suivant l'une quelconque des revendications précédentes et un support ou adjuvant pharmaceutiquement acceptables.

14. Utilisation d'un vecteur adénoviral suivant l'une quelconque des revendications 1 à 13 dans la production d'un médicament pour le traitement ou la prophylaxie d'une infection par le VIH.

15. Procédé pour la préparation d'un vecteur suivant l'une quelconque des revendications 1 à 13, comprenant les étapes : a) de fourniture d'un vecteur adénoviral ; b) de fourniture d'un plasmide portant les séquences d'antigènes du VIH liées de manière fonctionnelle à un promoteur convenable ; c) de transfection de cellules avec à la fois le plasmide et le vecteur ; d) d'écoulement d'un temps suffisant pour que la combinaison se produise ; et e) de récupération du vecteur viral recombinant portant les séquences d'antigènes du VIH.

16. Procédé pour amplifier une réponse immunitaire chez un mammifère, procédé qui comprend l'administration au mammifère d'une quantité convenable d'une composition immunogène suivant la revendication 13.

17. Protéine de fusion exprimée par le vecteur suivant l'une quelconque des revendications 1 à 12.

18. Protéine de fusion suivant la revendication 17, produite dans l'organisme humain.
